# EUROPEAN PATENT APPLICATION

(11) **EP 3 912 571 A1**
(43) Date of publication of application: **24.11.2021**
(21) Application number: 19883335.2
(22) Date of filing: 15.11.2019
(51) Int. Cl.: A61B 17/22, A61B 17/225, A61N 7/02, A61N 7/00

(54) **ULTRASONIC TRANSDUCER**

(30) Priority: 15.11.2018 KR 20180140429; 24.01.2019 KR 20190009447
(71) Applicant: Osteosys Co., Ltd., Seoul 08390 (KR)
(72) Inventor: OH, Jin Sik, Songpa-gu, Seoul 05681 (KR)
(74) Representative: Harden, Henry Simon
(86) International application number: PCT/KR2019/015629
(87) International publication number: WO 2020/101421

(57) **Abstract**

The present disclosure in some embodiments provides an ultrasonic transducer including a housing, a base disposed on a front surface of the housing, and multiple linear arrays each arranged in a radial direction on the base, extending from a central region of the base, and configured to irradiate a therapeutic ultrasonic wave, the linear arrays each including a plurality of piezoelectric elements which are linear elements extending side by side with each other in the radial direction.

## Description

### [Technical Field]

The present disclosure in some embodiments relates to an ultrasonic transducer.

### [Background Art]

The statements in this section merely provide background information related to the present disclosure and do not necessarily constitute prior art.

Extracorporeal shockwave therapy (ESWT) is a method of treating an affected area by irradiating therapeutic ultrasonic waves on an object to be examined.

Commonly-used ESWT apparatuses include an ultrasonic transducer and use a method of irradiating therapeutic ultrasonic waves from the ultrasonic transducer to an affected area.

In general, an ultrasonic transducer includes piezoelectric elements for irradiating therapeutic ultrasonic waves on the object.

At this time, for the treatment of the object, the ultrasonic transducer needs to have the piezoelectric elements in an arrangement that can effectively deliver the therapeutic ultrasound irradiation to the affected area.

In addition, to properly arrange the piezoelectric elements in a typical ultrasonic transducer so that the therapeutic ultrasonic waves can be efficiently delivered to the affected area of the object, the ceramic material for the piezoelectric elements needs to be processed into an appropriate shape.

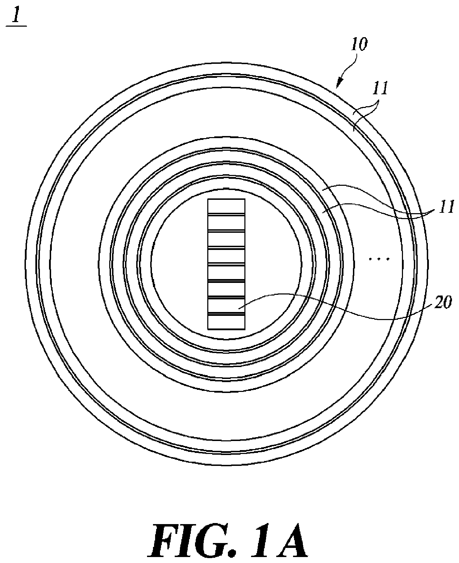

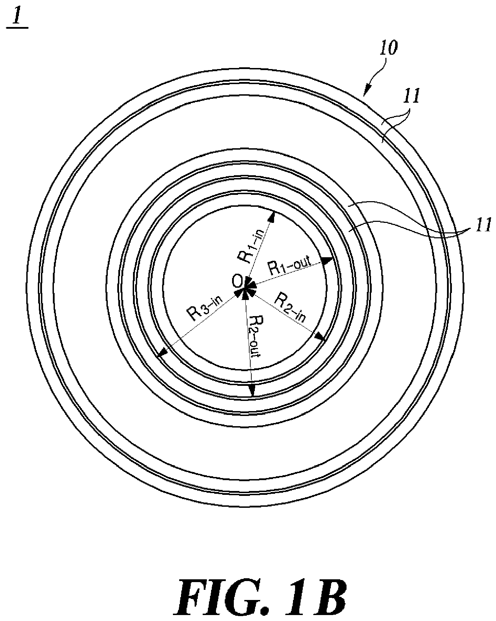

FIGS. 1A and 1B are front views of the configuration of a conventional ultrasonic transducer 1, including a ring-annular array.

As shown in FIG. 1, the ultrasonic transducer 1 has a piezoelectric multi-element array 10 including a plurality of piezoelectric elements 11 arranged in the form of ring-annular arrays and has an imaging probe 20. Whereas, it should be noted that FIG. 1B shows the ultrasonic transducer 1 with the imaging probe 20 omitted for convenience of description.

The piezoelectric elements 11 irradiate the therapeutic ultrasonic waves toward the affected area of the object.

The plurality of piezoelectric elements 11 are configured in circumferential shapes, that is, ring shapes, each having a different diameter, as shown in FIG. 1B.

Here, the therapeutic ultrasonic wave irradiated from each of the piezoelectric elements 11 may be irradiated intensively to the affected area of the object by arranging a separate acoustic lens (not shown).

Alternatively, adjustment of the amplitude or phase of the driving signal inputted to a separate piezoelectric element 11 can intensively irradiate the therapeutic ultrasonic wave to the affected area of the object.

On the other hand, if the innermost ring-shaped piezoelectric element 11 of the plurality of piezoelectric elements 11 has an inner diameter of Ri-in and an outer diameter of R₁₋ₒᵤₜ, then the second, larger diameter ring-shaped piezoelectric element 11 needs to be designed to have an inner diameter of R₂₋ᵢₙ which is equal to or slightly larger than R₁₋ₒᵤₜ.

Similarly, the third, larger diameter ring-shaped piezoelectric element 11 needs to be designed to have an inner diameter of R₃₋ᵢₙ which is equal to or slightly larger than R₂₋ₒᵤₜ.

Such an arrangement of the piezoelectric elements 11 in differently sized ring shapes as described above is to cause therapeutic ultrasonic waves emitted from the piezoelectric elements 11 to be effectively focused on the affected area of the object.

At this time, the piezoelectric elements 11 are manufactured by processing ceramic materials such as barium titanate (BaTiO₃), lead titanate (PbTiO₃), and lead zirconate system (PbZrO₃) by using a microelectromechanical systems (MEMS) technology.

In addition, there is a need for a process of attaching the piezoelectric elements 11 each having a differently sized ring shape on the substrate through a thermal pressing method or an ultrasonic bonding.

To manufacture the piezoelectric elements 11 made of ceramic materials to have different sizes in this way requires a special manufacturing facility capable of manufacturing respectively different diameters of ring-shaped piezoelectric elements 11 to go through a complicated manufacturing process.

Therefore, the ring-annular array type ultrasonic transducer 1 consumes a lot of effort to manufacture and increases the processing cost.

On the other hand, with a matrix array or a circular array type ultrasonic transducer other than the ring-annular array type shown in FIG. 1, there are difficulties in a manufacturing process similar to that described above.

Accordingly, there is a need to provide a proper arrangement of piezoelectric element arrays, which is easy to manufacture and has an appropriate therapeutic effect in the treatment of the subject.

### [Summary of Invention]

### [Technical Problem]

Therefore, the present disclosure seeks to provide an ultrasonic transducer having a simple configuration while having an excellent therapeutic effect due to the effective focusing of therapeutic ultrasonic waves on a target region.

Further, the present disclosure aims to provide an ultrasonic transducer that saves time and cost for processing or manufacturing.

### [Solution to Problem]

According to some embodiments of the present disclosure, an ultrasonic transducer is provided, including a housing, a base disposed on a front surface of the housing, and multiple linear arrays each arranged in a radial direction on the base, extending from a central region of the base, and configured to irradiate a therapeutic ultrasonic wave, the linear arrays each comprising a plurality of piezoelectric elements which are linear elements extending side by side with each other in the radial direction.

### [Brief Description of Drawings]

FIGS. 1A and 1B are front views of the configuration of a conventional ultrasonic transducer, including a ring-annular array.
FIG. 2 is a side view of the configuration of an ultrasonic transducer according to at least one embodiment of the present disclosure.
FIG. 3 is a front view of the configuration of a first aspect of the ultrasonic transducer according to at least one embodiment of the present disclosure.
FIG. 4 is a front view of the configuration of a second aspect of the ultrasonic transducer according to at least one embodiment of the present disclosure.
FIG. 5 shows the configuration of piezoelectric elements that constitute a component of the ultrasonic transducer according to at least one embodiment of the present disclosure.
FIGS. 6A and 6B show the configuration of an ultrasonic transducer according to another embodiment of the present disclosure.
FIGS. 7A to 7E show the performance test results of the ultrasonic transducer according to at least one embodiment of the present disclosure.
FIGS. 8A to 8E show the performance test results of a conventional ultrasonic transducer composed of ring-annular arrays.
FIG. 9 is a partial perspective view of an ultrasonic transducer according to yet another embodiment of the present disclosure.
FIG. 10 is a partial perspective view of the ultrasonic transducer of another embodiment of the present disclosure shown in FIGS. 6A and 6B for comparison with the embodiment of FIG. 9.
FIG. 11A shows a curvature profile of a linear array 930 as partially cut in a transverse direction D_{T} according to the yet another embodiment of the present disclosure.
FIG. 11B shows a curvature profile of a linear array 630 of another embodiment shown in FIG. 10 as cut in transverse direction D_{T}.
FIG. 12A is a measurement of the intensity of therapeutic ultrasonic waves in a subject when a focus is formed on a focus position at a reference focal length distanced from the ultrasound transducer according to the embodiment of FIG. 9.
FIG. 12B is a measurement of the intensity of the therapeutic ultrasonic waves from the ultrasound transducer according to another embodiment of FIG. 10.

### [Description of Embodiments]

Hereinafter, at least one embodiment of the present disclosure will be described in detail with reference to the accompanying drawings. In the following description, like reference numerals would rather designate like elements although the elements are shown in different drawings. Further, in the following description of the at least one embodiment, a detailed description of known functions and configurations incorporated herein will be omitted for the purpose of clarity and for brevity.

Additionally, various terms such as first, second, i), ii), (a), (b), etc., are used solely to differentiate one component from the other but not to imply or suggest the substances, order, or sequence of the components. Throughout this specification, when a part "includes" or "comprises" a component, the part is meant to further include other components, not excluding thereof unless there is a particular description contrary thereto.

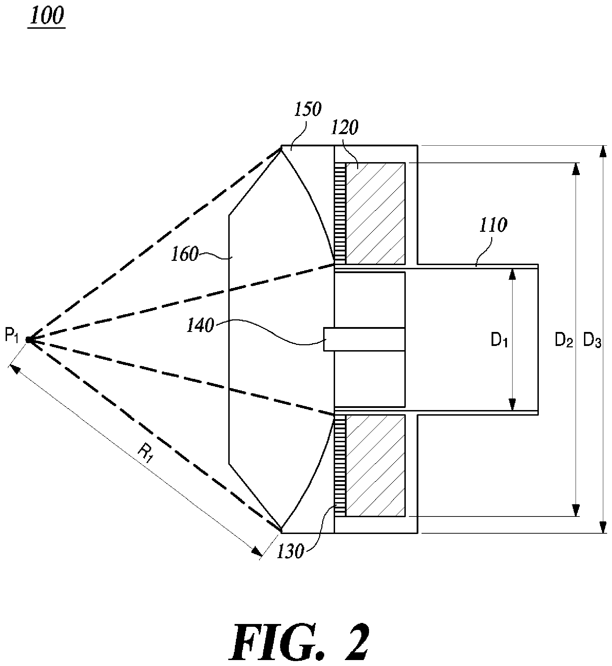

FIG. 2 is a side view of the configuration of an ultrasonic transducer 100 according to at least one embodiment of the present disclosure.

As shown in FIG. 2, the ultrasonic transducer 100 according to at least one embodiment of the present disclosure includes a housing 110, a base 120, one or more linear arrays 130, an imaging probe 140, an acoustic lens 150, and a gel pad 160.

The housing 110 provides a space for placement of the base 120, the linear arrays 130, the imaging probe 140, and others.

Of the housing 110, an area in which the base 120, the linear arrays 130, and the imaging probe 140 are placed may be referred to as a front portion which may then be peripherally bent forward into an L or "¬ " shape so that the front portion of the housing 110 has a space for the base 120.

In addition, the front portion of the housing 110 may be formed in an exemplary cylindrical or polygonal column shape. Disposed on the front portion of the housing 110 is the base 120 having an upper surface, on which the linear arrays 130 are disposed.

In addition, disposed on the housing 110 centrally and forwardly thereof is the imaging probe 140.

On the other hand, the housing 110 shown in FIG. 2 is configured to have an outer diameter D₃ of 110mm.

In addition, each linear array 130 extends to a position spaced 5mm from the outermost of the housing 110, and two diagonally opposite linear arrays 130 have their distal ends separated by a distance D₂ of 100mm.

The base 120 provides a space for the linear arrays 130 to be disposed. The linear array 130 are disposed on the front surface of the base 120 which may serve as a backing panel supporting the linear arrays 130.

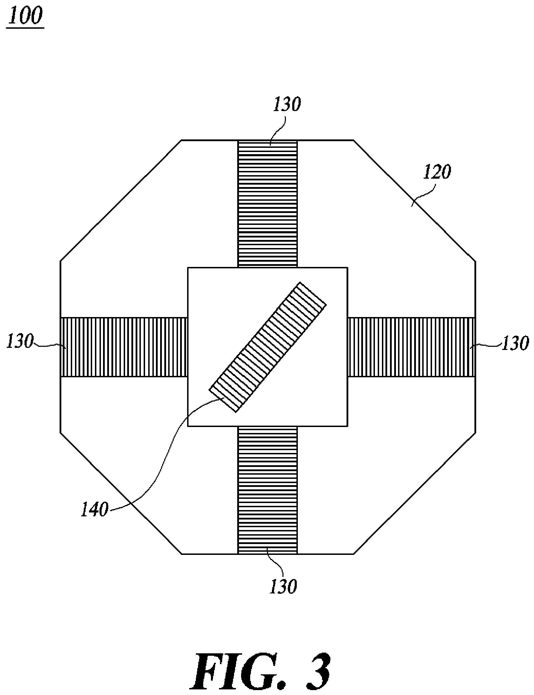

As shown in FIG. 3, the base 120 may be formed in an octagonal shape as shown in FIG. 3, which is only exemplary and may have other shapes, such as a circular shape.

Disposed centrally of the base 120 is the imaging probe 140, and the linear arrays 130 are disposed circumferentially of a region in which the imaging probe 140 is disposed. In the ultrasonic transducer 100 shown in FIG. 2, the region in which the imaging probe 140 is disposed is designed to have a width D₁ of about 42 mm.

The linear array 130 has its piezoelectric elements that generate vibrations which in turn generate therapeutic ultrasonic waves that are irradiated toward the object.

The linear array 130 refers to a linear array type in which rectilinear piezoelectric elements having similar lengths and widths are arranged side by side and adjacent to each other. The linear array 130 includes long sides formed to be relatively elongated along the longitudinal direction thereof, and short sides formed to be relatively short along the latitudinal or widthwise direction thereof.

Multiples of the linear array 130 are arranged over the front surface of the base 120. The multiple linear arrays 130 are disposed adjacent to the central region of the front surface of the base 120, and the linear arrays 130 are radially disposed around the central region of the front surface of the base 120.

Specifically, a radial direction of the base 120 may refer to an arbitrary direction from the center point of the front surface of the base 120 toward the outer edge of the front surface of the base 120, wherein the linear array 130 is arranged so that its longitudinal axis lies on the radial direction of the base 120.

At this time, the long sides of the linear array 130 are disposed in parallel to the radial direction of the base 120, and the short sides of the linear array 130 are vertically disposed to the radial direction of the base 120.

On the other hand, each linear array 130 is composed of a plurality of piezoelectric elements, and due to such a configuration, the ultrasonic transducer 100 according to at least one embodiment of the present disclosure may be made advantageously through a simple manufacturing process and uncomplicated facilities for manufacturing as will be described in detail below.

The imaging probe 140 serves to generate information about the ultrasound image of the object and to transfer the same information to an unshown display unit. Although not shown, the imaging probe 140 includes an imaging ultrasound transmitter and an imaging ultrasound receiver.

The imaging ultrasound transmitter irradiates imaging ultrasonic waves toward an object. The imaging ultrasound receiver receives an echo signal of the imaging ultrasonic waves reflected by the object.

The echo signal received by the imaging ultrasound receiver is converted into an image signal by an unshown signal processing unit, and the image signal is transmitted to the display unit for outputting the ultrasonic image of the object.

The acoustic lens 150 concentrates therapeutic ultrasonic waves into a target region.

The acoustic lens 150 is disposed forwardly of the base 120 and the plurality of linear arrays 130, and the therapeutic ultrasound component irradiated from the linear arrays 130 passes through the acoustic lens 150 to be focused on one area of the object.

The acoustic lens 150 provided as described above can efficiently focus the therapeutic ultrasonic waves, resulting in an enhanced curative effect.

On the other hand, at least one embodiment of the present disclosure illustrates the configuration having the acoustic lens 150 for focusing the therapeutic ultrasonic waves, although the one embodiment further includes a configuration for adjusting the focusing position of the therapeutic ultrasonic waves by using a beamforming technique.

In particular, the one embodiment of the present disclosure may still include a configuration in which the focusing position of the therapeutic ultrasonic waves is adjusted by adjusting the amplitude or phase of the driving signal inputted to the piezoelectric elements of the linear array 130. In this case, the acoustic lens 150 is not necessarily provided for concentrating therapeutic ultrasonic waves.

On the other hand, since each therapeutic ultrasonic wave needs to be focused on a set focal region P₁, the acoustic lens 150 may be configured so that the degree of refraction of each therapeutic ultrasound component is different.

Meanwhile, the acoustic lens 150 may have a curvature R₁ of, for example, 90 mmR, although and the acoustic lens 150 may be designed to have a different, appropriate curvature.

The gel pad 160 is disposed on the front of the acoustic lens 150 to facilitate the delivery of the therapeutic ultrasonic waves. The therapeutic ultrasound waves irradiated from the linear array 130 are focused by the acoustic lens 150 and passes through the gel pad 160 to be irradiated through the object.

The gel pad 160 may be internally filled with fluid and may serve to reduce attenuation, scattering, and the like of therapeutic ultrasonic waves irradiated from the linear array 130.

In addition, once filled with a fluid, the gel pad 160 may also serve to cool the heat generated by ultrasonic waves.

The thickness of the gel pad 160 from the base 120 may be about 30 mm, although the thickness thereof may be designed differently.

In addition, the ultrasonic transducer 100 according to at least one embodiment of the present disclosure may include unshown signal transmission/reception lines inside the housing 110 or in an unshown controller.

In addition, the ultrasonic transducer 100 according to at least one embodiment of the present disclosure may include features such as an unshown power button and an operation input button capable of controlling its operation, and it may be linked to a separate control device. However, those features are not to be focused on in the configuration of the present disclosure, and a detailed description thereof will be omitted.

FIG. 3 is a front view of the configuration of a first aspect of the ultrasonic transducer 100 according to at least one embodiment of the present disclosure.

As showing in FIG. 3, the first aspect of the ultrasonic transducer 100 according to at least one embodiment of the present disclosure is configured to include four linear arrays 130 arranged on the base 120 along the radial direction of the base 120.

The ultrasonic transducer 100 according to at least one embodiment of the present disclosure has a distinctive type in which multiple uniformly rectilinear piezoelectric elements are arranged adjacent to each other into a linear array type. Accordingly, each linear array 130 has a rectangular shape.

The at least one embodiment of the present disclosure illustrates a case where the linear array 130 has an aspect ratio of short side to long side of about 16 mm to 34 mm. The at least one embodiment may be modified to have other aspect ratios of the linear array 130.

At this time, in consideration of the treatment efficiency by the degree of focusing the therapeutic ultrasonic waves when the ultrasonic transducer 100 is operated and of the arrangement and shape of the base 120 and other components, the aspect ratio of short side to long side is preferably 1:2 to 1:4 or less.

The linear arrays 130 constituting the first aspect of ultrasonic transducer 100 are disposed interspaced from one another at a predetermined angle, respectively. At this time, the angle at which each linear array 130 is spaced from the adjacent one may be about 90 degrees.

Accordingly, the linear arrays 130 are disposed to have a length in the upper, lower, left and right directions surrounding the center of the base 120, respectively, and the respective linear arrays 130 are formed to have the same shape.

Therefore, the respective linear arrays 130 have a point-symmetrical arrangement relative to the center of the base 120.

The respective linear arrays 130 may be fabricated to have a uniform shape and size. Therefore, in manufacturing the ultrasonic transducer 100, there is no need for a manufacturing facility or the like for separately manufacturing each array or each piezoelectric element.

Accordingly, the ultrasonic transducer 100 according to at least one embodiment of the present disclosure takes a simple process of manufacturing the respective linear arrays 130 as will be detailed below, and it obviates the need for such a complicated process and a manufacturing facility as in the comparative example described above to manufacture the linear arrays 130.

On the other hand, the present applicant was able to confirm that the first aspect of the ultrasonic transducer 100 according to the at least one embodiment has a significant efficiency in terms of its therapeutic effect when compared to the aforementioned comparative example.

Further, despite allocating a narrower area to its linear arrays 130 than the annular array type ultrasonic transducer 1 does, the ultrasonic transducer 100 according to the at least one embodiment was confirmed to have the curative effect similar to that of the annular array type ultrasonic transducer 1.

This provides an advantage that the ultrasonic transducer 100 according to the at least one embodiment can be manufactured more simply, consuming less material than the comparative example as well as perform an effective treatment on a subject.

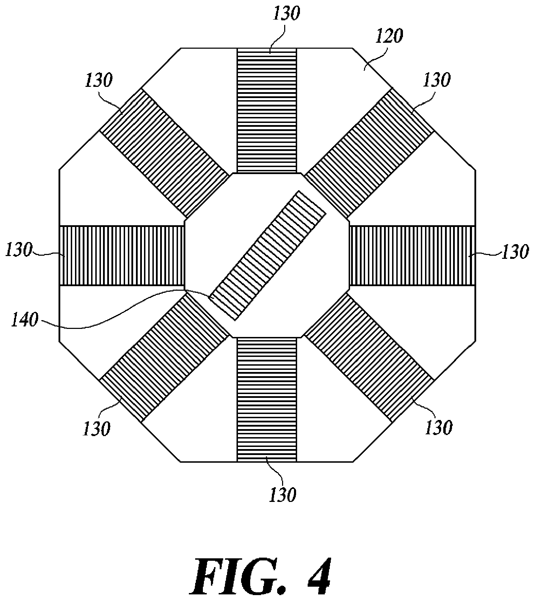

FIG. 4 is a front view of the configuration of a second aspect of the ultrasonic transducer 100 according to at least one embodiment of the present disclosure.

The second aspect of the at least one embodiment of the present disclosure, as in the case of the first aspect, a plurality of line-type linear arrays 130 are disposed on the base 120. Specifically, the linear arrays 130 are disposed adjacent to the central region of the base 120 so that the linear arrays 130 have their longitudinal axes extend in the radial direction of the base 120.

However, different from the first aspect, the second aspect has the linear arrays 130 spaced apart from each other by 45 degrees. Therefore, in contrast to the first aspect wherein a high-intensity focused ultrasound (HIFU) transducer unit is composed of a total of four linear arrays 130, the second aspect provides a HIFU transducer unit composed of a total of eight linear arrays 130.

In this way, the second aspect doubles the number of linear arrays 130 of the first aspect to minimize the difference in the area occupied by the linear arrays 130 on the base 120 when compared with the annular array type transducer.

This can increase the area of the irradiation source for irradiating the therapeutic ultrasonic waves, thereby increasing the ultrasound focusing and curative effect to a level almost the same as the annular array type transducer.

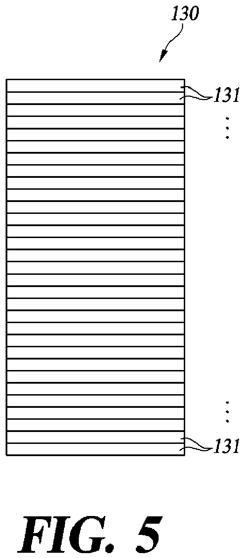

FIG. 5 shows the configuration of piezoelectric elements that constitute a component of the ultrasonic transducer 100 according to at least one embodiment of the present disclosure.

As shown in FIG. 5, the at least one embodiment of the present disclosure has the linear array 130 made of a plurality of piezoelectric elements.

At this time, the piezoelectric elements may be manufactured using a piezoelectric material such as lead zirconate titanate (PZT), polyvinylidene fluoride (PVDF), lead magnesium niobate (PMN), and they may be electrically connected to an unshown printed circuit board wherein the driving signal establishes the piezoelectric effect for generating therapeutic ultrasonic waves.

At this time, the plurality of piezoelectric elements are each made in a linear form, and each piezoelectric element may have the same shape and size.

Therefore, during the manufacture of the linear array 130 in the at least one embodiment, the multiple piezoelectric elements may be designed to undergo the same fabrication process.

During manufacture, different from the annular array type transducer which involves fabricating and placing individual devices having different diameters, the linear array 130 in the at least one embodiment obviates the need for complicated manufacturing equipment and manufacturing processes.

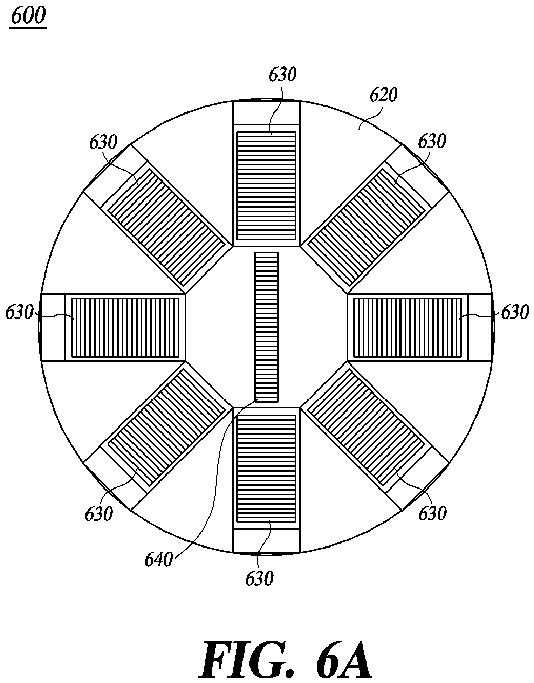

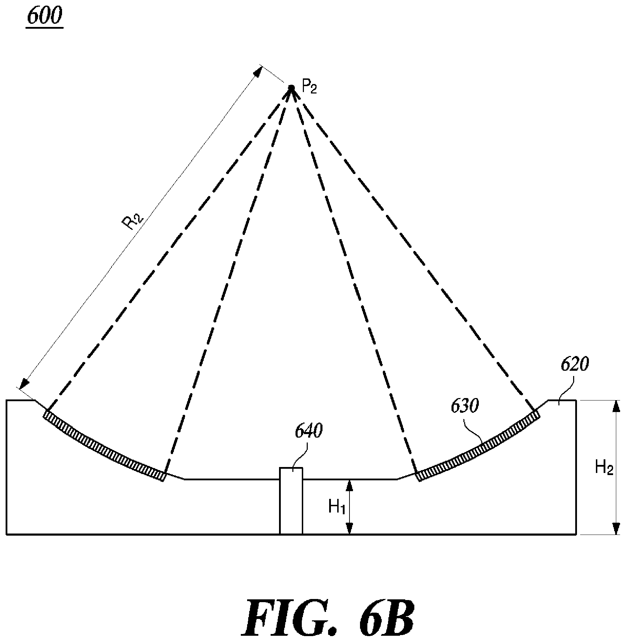

FIGS. 6A and 6B show the configuration of an ultrasonic transducer 600 according to another embodiment of the present disclosure.

Specifically, FIG. 6A is a front view of the ultrasonic transducer 600 according to another embodiment of the present disclosure, and FIG. 6B is a side view of the ultrasonic transducer 600 according to the another embodiment.

The ultrasonic transducer 600 according to the another embodiment includes the configuration of the ultrasonic transducer 100 according to the at least one embodiment as long as they are not contrary to each other.

However, different from the ultrasonic transducer 100 according to the at least one embodiment, the ultrasonic transducer 600 according to the another embodiment has linear arrays 630 disposed on a curved surface.

As shown in FIGS. 6A and 6B, the ultrasonic transducer 600 according to the another embodiment has a base 620 which is formed to have predetermined parabolic curved portions over the outer region except for the central region thereof, to face the area irradiated with therapeutic ultrasonic waves.

At this time, the central region of the base 620 may be formed to be constant in, for example, its thickness and may be provided with an imaging probe 640. Meanwhile, in the another embodiment, the central portion of the base 620 has a height H₁ of about 12.4 mm.

The front portion of the base 620 needs to be curved toward the area to be irradiated with therapeutic ultrasonic waves, for which the thickness of the base 620 gradually increases as it deviates from the central region of the base 620. For example, the base 620 has a height H₂ of about 30 mm at the outermost portion thereof.

At this time, the curvature of the front portion of the base 620 may be determined by roughly considering the distance from the base 620 to the focal position P₂ of the therapeutic ultrasonic waves, and illustratively, the front portion of the base 620 has a curvature R₂ of 90 mmR.

In addition, conforming to such curved configuration of the front portion of the base 620, the respective piezoelectric elements lie flush with the same curvature surface of the base 620. Accordingly, the respective linear arrays 630 are formed on the same curvature surface.

This can increase the efficiency of focusing the therapeutic ultrasonic waves irradiated from the respective piezoelectric elements into the single area of the object.

As described above, the another embodiment of the present disclosure, which has the respective piezoelectric elements formed on the curvature surface, can have a high focusing effect of therapeutic ultrasonic waves even without the acoustic lens 150 (see FIG. 2) unlike the ultrasonic transducer 100 according to the at least one embodiment.

Accordingly, the ultrasonic transducer 600 according to the another embodiment can be manufactured save the process of manufacturing and attaching the acoustic lens 150 (see FIG. 2), thus simplifying the manufacturing process and manufacturing equipment to save the manufacturing cost.

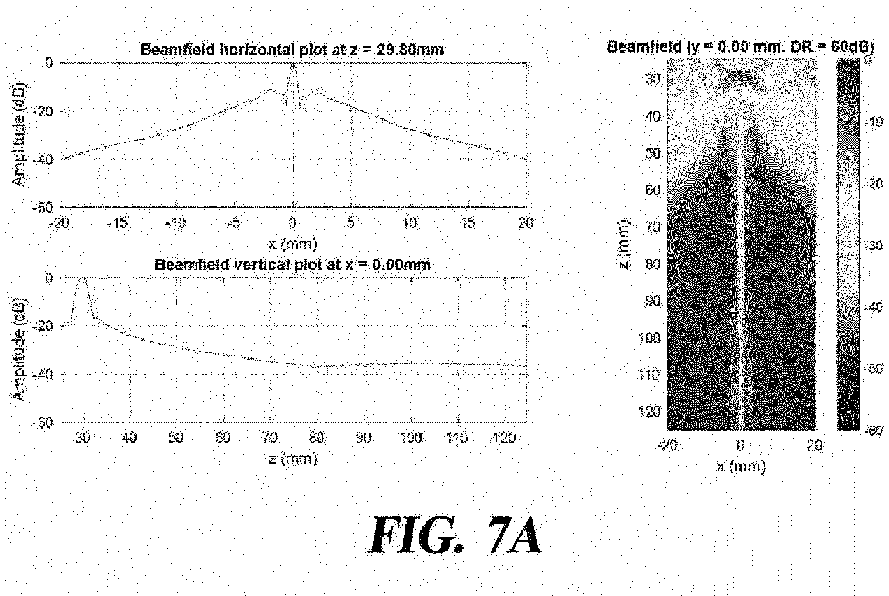

FIGS. 7A to 7E show the performance test results of the ultrasonic transducer 100 according to at least one embodiment of the present disclosure.

At this time, the ultrasonic transducer 100 was tested using a configuration in which eight linear arrays 130 are disposed on the base, and detailed specifications of the ultrasonic transducer 100 are as described above.

Here, FIG. 7A is a measurement of the intensity of a therapeutic ultrasonic wave on a subject when a focus is formed on a focus position at a reference focal length distanced from the ultrasound transducer 100 according to the at least one embodiment. Meanwhile, for convenience of description, the focal length at this time is set to 0 mm.

In addition, the z-axis direction signifies the depth direction of the object, and the x-axis and y-axis directions signify the horizontal axis direction and the vertical axis direction perpendicular to the horizontal axis direction respectively at the same depth in the object.

On the other hand, the test was conducted by providing the therapeutic ultrasonic wave with a frequency of 1.5 MHz, and the dynamic range (DR) was set to be 60 dB. These settings apply to all of the following processes.

In addition, depending on the intensity of the therapeutic ultrasonic wave in the subject, the region with the strongest therapeutic ultrasonic wave was arranged to be displayed in red, and the region that has the weakest therapeutic ultrasonic wave was displayed in blue.

First, the lower-left graph of FIG. 7A shows the intensity of therapeutic ultrasonic wave dependent on depth at the point where the x-axis coordinate is 0, that is, the point where the strongest focused intensity is on the x-y plane. At this time, the amplitude of the therapeutic ultrasonic wave is the largest at a point having a depth of about 30 mm from the surface of the object, and thus the therapeutic ultrasonic wave exhibits the greatest intensity at this point.

The upper left graph of FIG. 7A shows the intensity of therapeutic ultrasonic waves according to a change in position in the x-axis direction at a depth of 29.80 mm in the object. At this time, it can be seen that where the x-axis coordinate is 0, the intensity of the therapeutic ultrasonic wave is the largest, and the intensity thereof is symmetrically decreased as it is further away from the x-axis coordinate 0.

In addition, as shown in the right graph of FIG. 7A, graphs are formed radially around a point having a depth of about 30 mm in the object. Since the focusing point of the therapeutic ultrasonic waves is located at a point not deep in the object, it can be seen that the therapeutic ultrasonic waves are effectively focused at that point.

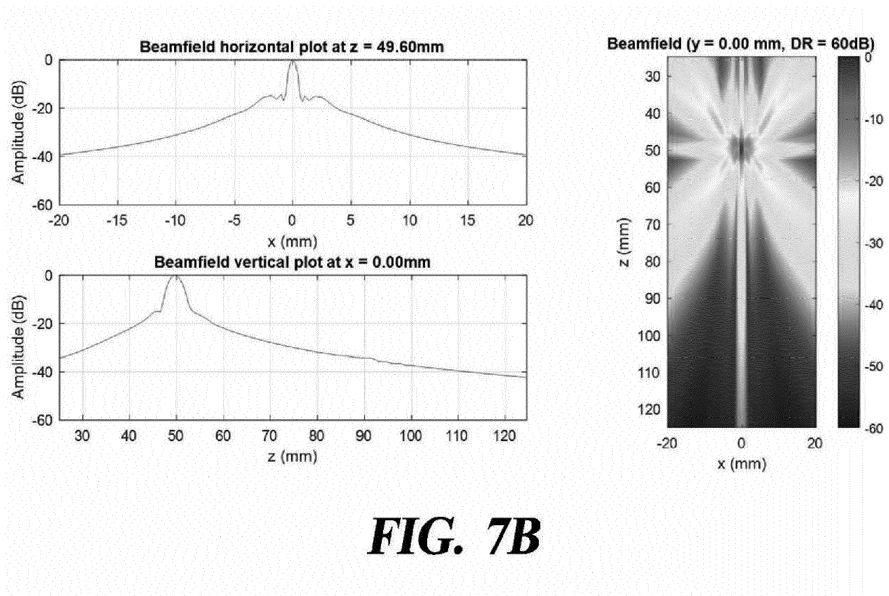

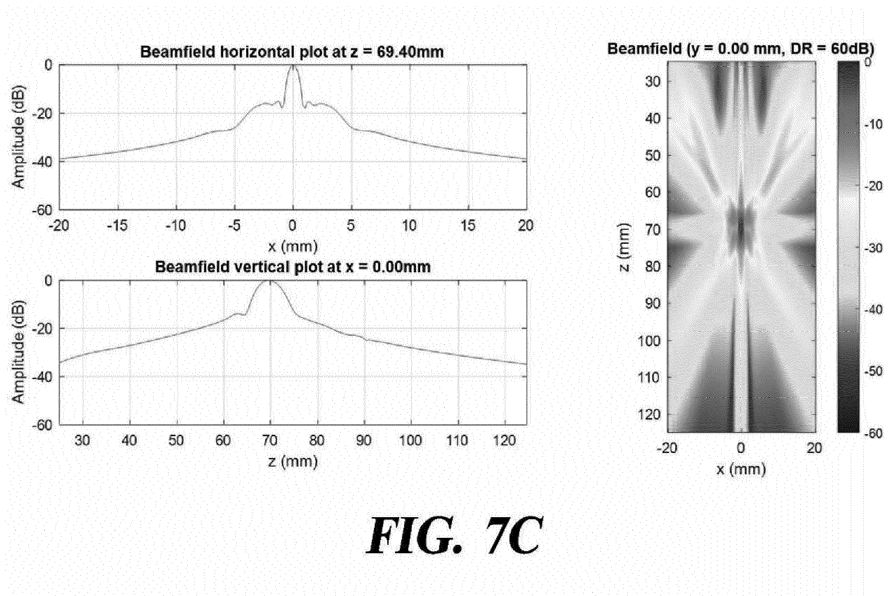

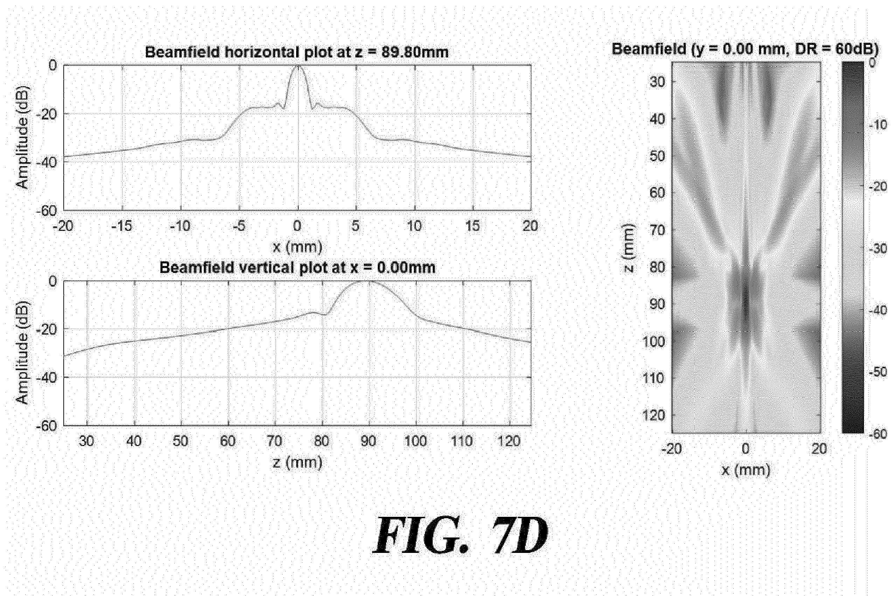

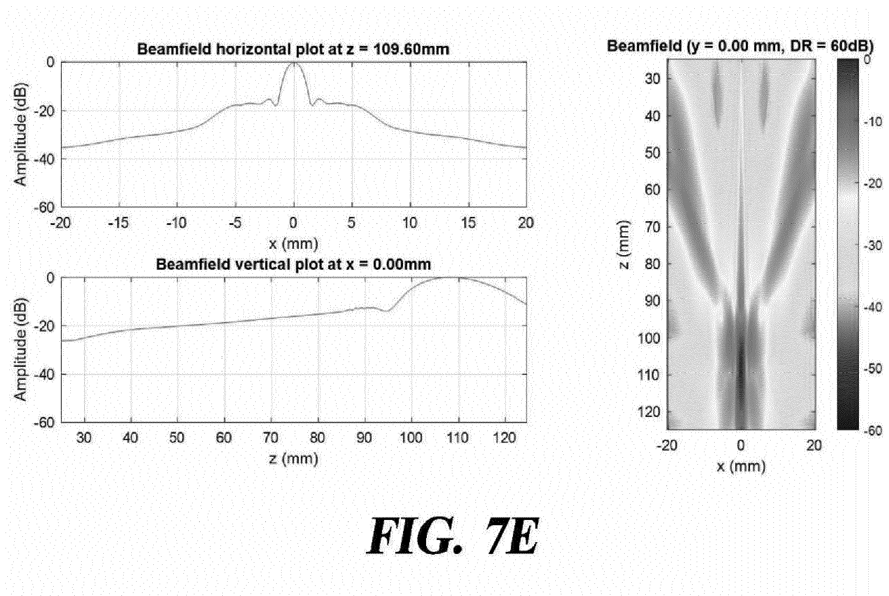

FIGS. 7B, 7C, 7D, and 7E respectively show the intensities of a therapeutic ultrasonic wave at a position in the object when the focal position is 20 mm, 40 mm, 60 mm, and 80 mm from the reference focus.

Different focal positions of the therapeutic ultrasonic waves from that of FIG. 7A establish different positions in which the therapeutic ultrasonic waves are most strongly focused. In particular, FIGS. 7B, 7C, 7D, and 7E exhibit that the strongest therapeutic ultrasonic waves are focused at depths of 49.60 mm, 69.40 mm, 89.80 mm, and 109.60 mm in the object, respectively.

In FIGS. 7B to 7E, the therapeutic ultrasound waves are focused in radial forms similar to the case of FIG. 7A. However, the radial focus intensity decreases from FIG. 7A to FIG. 7E because the transfer efficiency of therapeutic ultrasonic waves gradually decreases toward the deeper position of the subject.

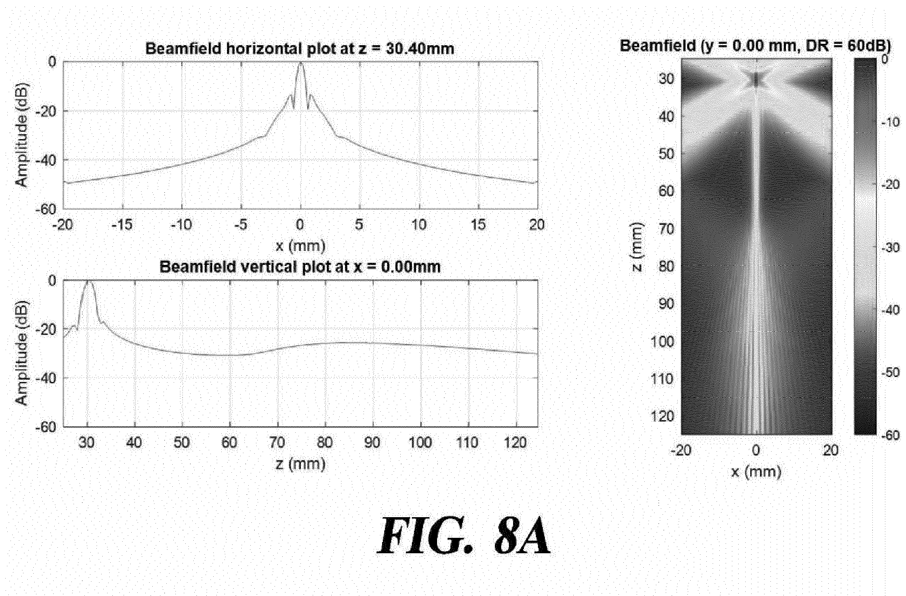

FIGS. 8A to 8E show the performance test results of the conventional ultrasonic transducer 1 composed of ring-annular arrays.

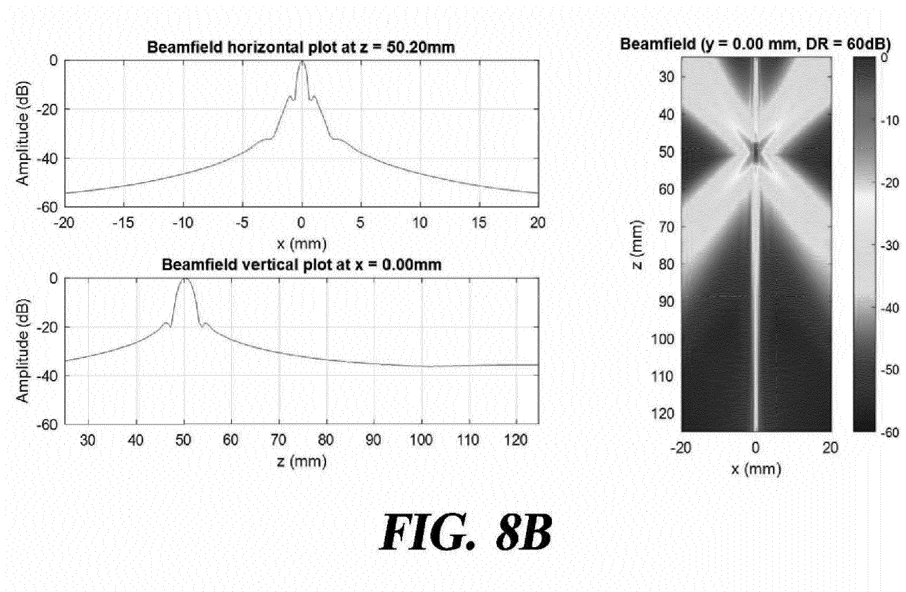

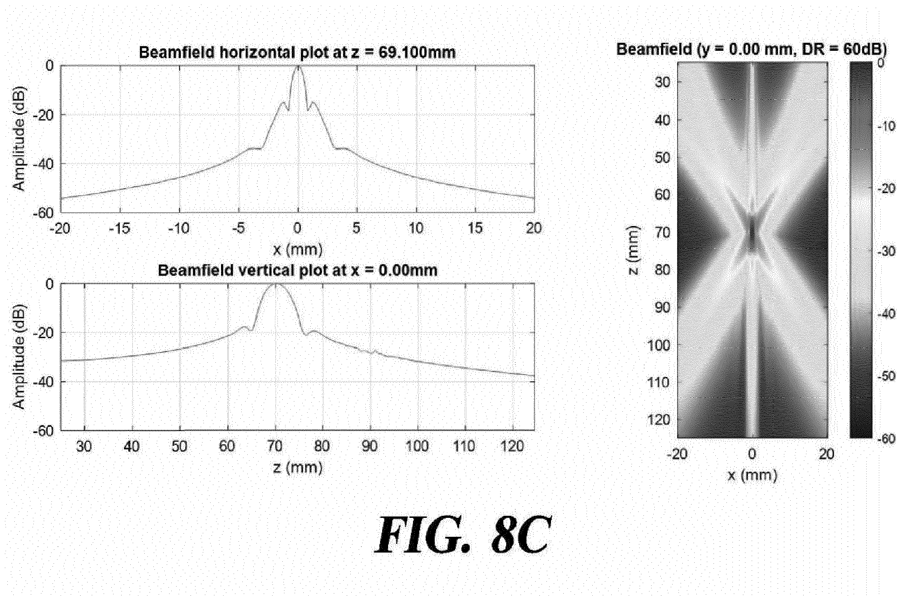

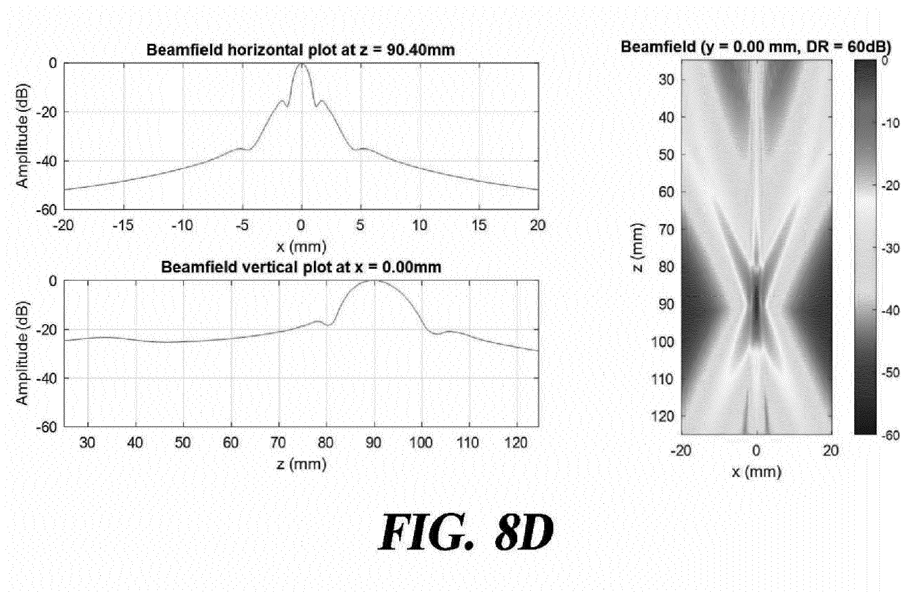

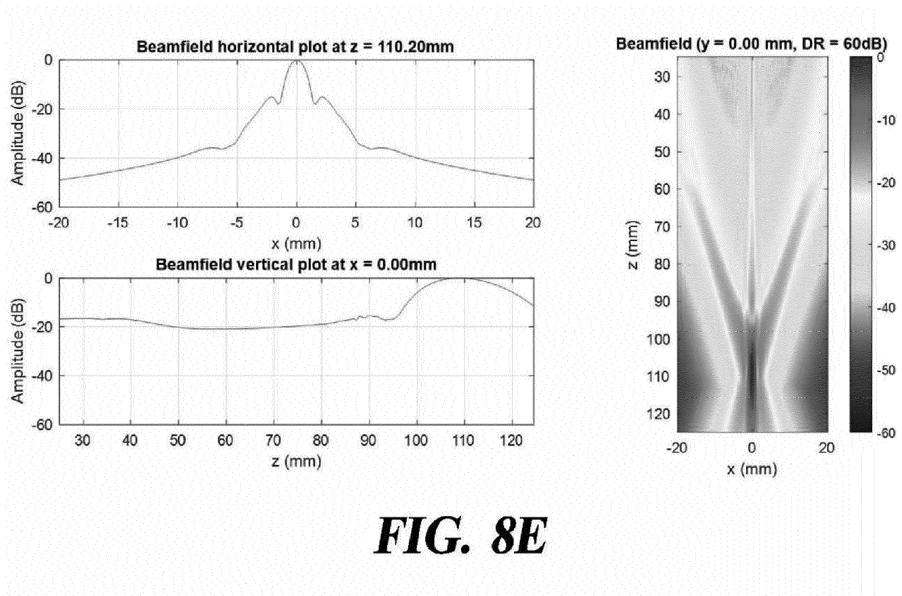

Specifically, FIGS. 8A, 8B, 8C, 8D, and 8E show the ultrasound intensities in the object positions when the focal positions of the therapeutic ultrasound waves are 0 mm, 20 mm, 40 mm, 60 mm, and 80 mm, respectively.

The performance test was performed using the typical transducer 1, as shown in FIG. 1. The transducer 1 has the ring-annular array 10 composed of a total of twenty-four ring-annular arrays of the piezoelectric elements 11 each having a thickness of 1.2 mm.

A comparison between FIG. 7 and FIG. 8 reveals that the performance graphs of the ultrasonic transducer 100 according to the at least one embodiment shown in FIG. 7 are quite similar in shape to those of the ring-annular array type ultrasonic transducer shown in FIG 8.

In other words, FIGS. 7A and 8A, FIGS 7B and 8B, FIGS 7C and 8C, FIGS 7D and 8D, and FIGS. 7E and 8E are very similar to each other in shape, and they show no significant difference in the strength of the therapeutic ultrasonic waves in the focused area.

This signifies that for the smaller footprint of the linear transducer arrays 130 of the ultrasonic transducer 100 than that of the ring-annular arrays of the ultrasonic transducer 1, the ultrasonic transducer 100 yet provides the focus intensity and strength of the therapeutic ultrasonic waves as good as that of the ultrasound transducer 1 shown in FIG. 1.

Therefore, it can be seen that the ultrasonic transducer 100 of the present disclosure has a superior curative effect while being simple to manufacture compared to the prior art transducer 1.

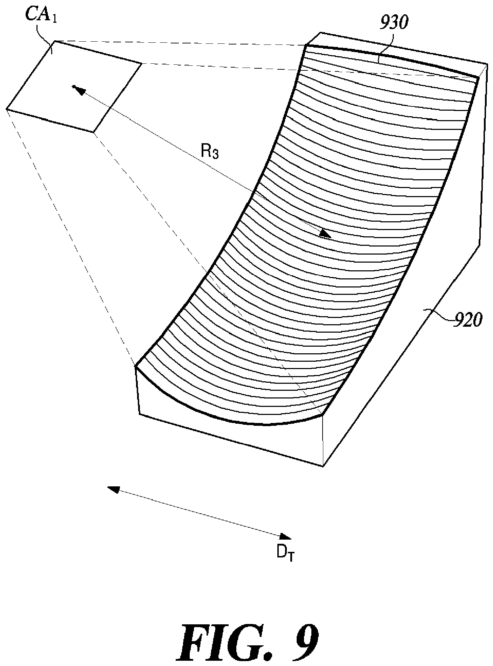

FIG. 9 is a partial perspective view of an ultrasonic transducer according to yet another embodiment of the present disclosure.

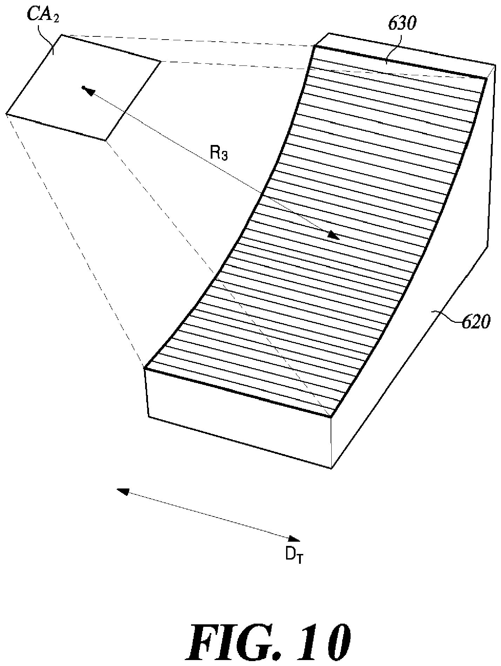

FIG. 10 is a partial perspective view of the ultrasonic transducer of the another embodiment of the present disclosure shown in FIGS. 6A and 6B for comparison with the yet another embodiment of FIG. 9.

The following will concentrate on differences between the yet another embodiment shown in FIG. 9 and the aforementioned embodiment in FIGS. 6A and 6B, and repeated description will be omitted.

As shown in FIGS. 9 and 10, the yet another embodiment in FIG. 9 provides an ultrasonic transducer having a linear array 930 with a distinctive curvature that is bi-directional, i.e., in the longitudinal direction and transverse direction D_{T}, as compared to the another embodiment illustrated in FIG. 10.

Accordingly, in the yet another embodiment of FIG. 9, the ultrasonic transducer has a base 920 that is at least partially curved in cross section in both the longitudinal and transverse directions, conforming to the curved surface defined by the linear array 930.

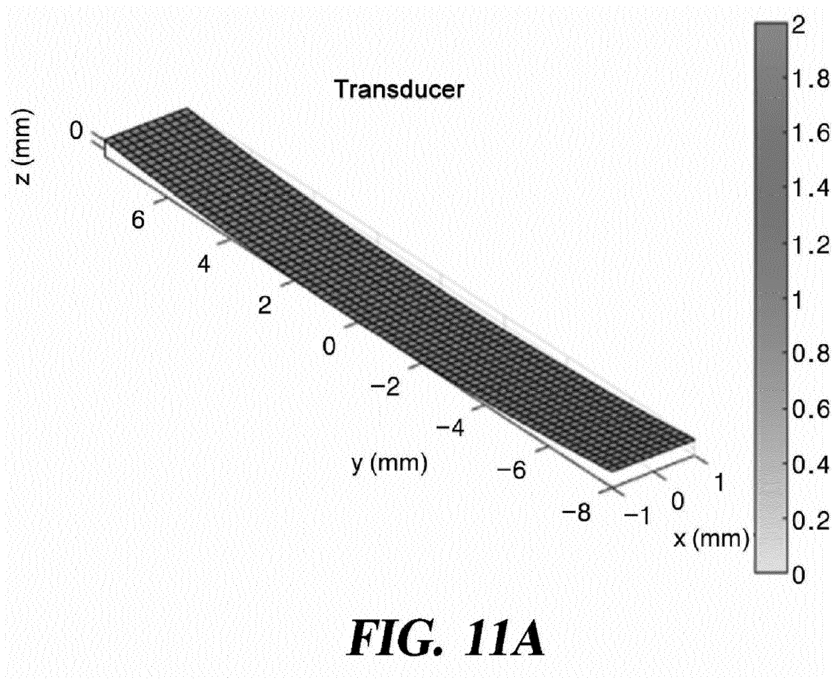

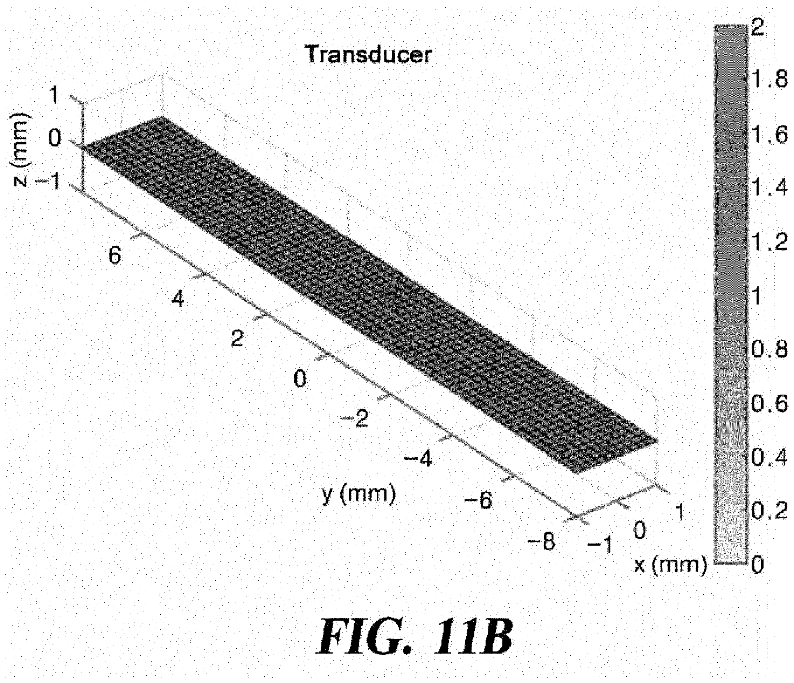

Referring to FIGS. 11A and 11B for comparing profiles formed in transverse direction D_{T}, FIG. 11A illustrates a curvature profile of the linear array 930 as partially cut in transverse direction D_{T} according to the yet another embodiment of the present disclosure, and FIG. 11B shows a curvature profile of the linear array 630 of the another embodiment shown in FIG. 10 as cut in transverse direction D_{T}.

As shown, the linear array 930 illustrated in FIG. 11A has a curvature in which a height varies in the transverse direction, while the linear array 630 illustrated in FIG. 11B forms a plane without a height difference in the transverse direction.

Referring back to FIGS. 9 and 10, the linear array 930 of FIG. 9 and the linear array 630 of FIG. 10 have different ultrasound collecting performances due to their difference in the curvature formed along transverse direction D_{T}.

For example, as illustrated in FIG. 9, the linear array 930 may be interpreted as irradiating ultrasonic waves to be concentrated at an ultrasound collection surface CA₁ distanced from the linear array 930 by an arbitrary radius R3.

In addition, as illustrated in FIG. 10, the linear array 630 may be interpreted as irradiating ultrasonic waves to be concentrated at an ultrasound collection surface CA₂ distanced from the linear array 630 by the same radius R₃ as illustrated in FIG. 9.

At this time, ultrasound collection surface CA₁ of FIG. 9 may be smaller than ultrasound collection surface CA₂ of FIG. 10 by approximately 20% in the illustrated embodiments. This means that assuming that the distance to the patient's affected area is determined as R₃, an ultrasound pressure of 20% can be further increased by a transducer of the same size.

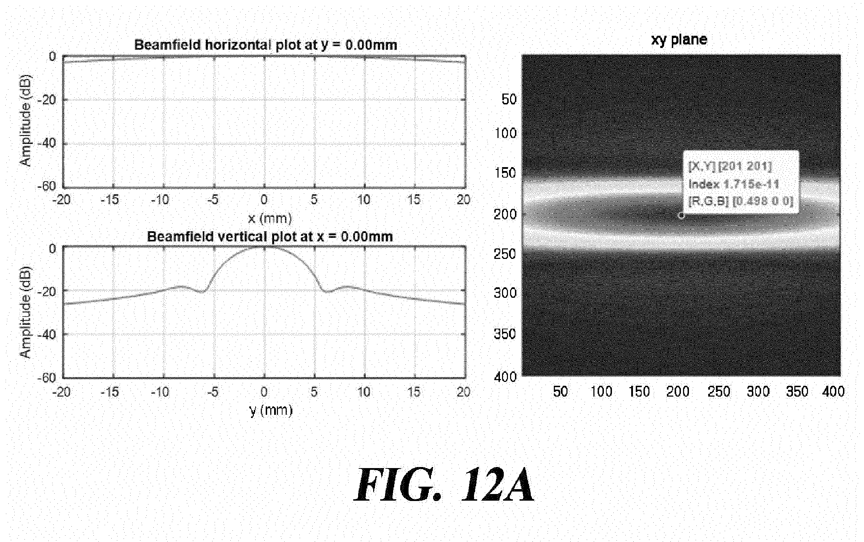

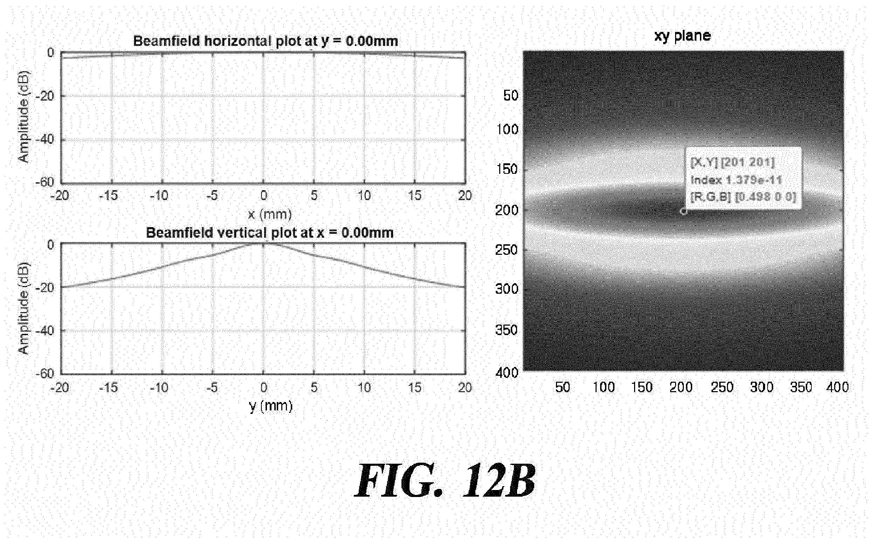

In particular, FIG. 12A is a measurement of the intensity of therapeutic ultrasonic waves in a subject with a focus formed on a focus position at a reference focal length distanced from the ultrasound transducer according to the yet another embodiment of FIG. 9, and FIG. 12B is a measurement of the intensity of therapeutic ultrasonic waves from the ultrasound transducer according to the another embodiment of FIG. 10.

As shown in FIG. 12B, the other embodiment of FIG. 10 shows an aspect in which the intensity of the vertical beamfield gradually increases as the focal length approaches 0 mm. In contrast, as illustrated in FIG. 12A, the yet another embodiment of FIG. 9 shows a tendency that the intensity of the vertical beamfield is rapidly increased as the focal length approaches 0 mm, and thus the beam intensity is concentrated near the focal length of 0 mm.

As described above, since the ultrasonic transducers 100, 600 according to the respective embodiments of the present disclosure are manufactured by using the line-type linear arrays 130, 630, they are manufactured through simplified manufacturing process by advantageously utilizing simplified manufacturing equipment, as compared to using other types of arrays.

In addition, the ultrasonic transducers 100, 600 according to the respective embodiments of the present disclosure have the above manufacturing advantages and yet have excellent therapeutic effects as with the prior art ultrasonic transducers.

Although exemplary embodiments of the present disclosure have been described for illustrative purposes, those skilled in the art will appreciate that various modifications, additions, and substitutions are possible, without departing from the idea and scope of the claimed invention. Therefore, exemplary embodiments of the present disclosure have been described for the sake of brevity and clarity. The scope of the technical idea of the present embodiments is not limited by the illustrations. Accordingly, one of ordinary skill would understand the scope of the claimed invention is not to be limited by the above explicitly described embodiments but by the claims and equivalents thereof.

### REFERENCE SIGNS LIST

| | | | |
|---|---|---|---|
| 100: | ultrasonic transducer | 110: | housing |
| 120: | base | 130: | linear array |
| 140: | imaging probe | 150: | acoustic lens |
| 160: | gel pad | | |

## Claims

1. An ultrasonic transducer, comprising:
a housing;
a base disposed on a front surface of the housing; and
multiple linear arrays each arranged in a radial direction on the base, extending from a central region of the base, and configured to irradiate a therapeutic ultrasonic wave, the linear arrays each comprising a plurality of piezoelectric elements which are linear elements extending side by side with each other in the radial direction.

2. The ultrasonic transducer of claim 1, wherein the multiple linear arrays are spaced apart to have a certain angle with respect to each other.

3. The ultrasonic transducer of claim 2, wherein the multiple linear arrays are oriented at a right angle with respect to each other.

4. The ultrasonic transducer of claim 2, wherein the multiple linear arrays are oriented at an angle of 45 degrees with respect to each other.

5. The ultrasonic transducer of claims 3 and 4, wherein the multiple linear arrays are arranged to be symmetrical with respect to each other around the central region of the base.

6. The ultrasonic transducer of claim 1, wherein the base has a front surface that assumes a parabolic shape, and the multiple linear arrays are disposed on the base and arranged along a curvature conforming to the parabolic shape of the front surface of the base.

7. The ultrasonic transducer of claim 1, wherein the multiple linear arrays each assumes a rectangular shape having a long side extending parallel to the radial direction and a short side perpendicular to the long side, and the multiple linear arrays each have an aspect ratio of long side to short side of 2:1 to 3:1.

8. The ultrasonic transducer of claim 1, further comprising:
an imaging probe disposed in the central region of the base and configured to transmit and receive imaging ultrasonic waves to and from an object.

9. The ultrasonic transducer of claim 1, wherein the piezoelectric elements are obtained by processing a ceramic material.

10. The ultrasonic transducer of claim 1, wherein the base at least partially includes a curved surface having a curvature in both a first direction and a second direction perpendicular to the first direction, and the multiple linear arrays are disposed on the curved surface and arranged along the curvature in both the first direction and the second direction.
